# EUROPEAN PATENT APPLICATION

(11) **EP 4 574 007 A1**
(43) Date of publication of application: **25.06.2025**
(21) Application number: 23220055.0
(22) Date of filing: 22.12.2023
(51) Int. Cl.: A61B 1/00, A61B 1/04, A61B 18/00, A61N 5/067

(54) **MEDICAL CAMERA SYSTEM AND METHOD FOR CAPTURING IMAGES AND PROCESSING SAID IMAGES**

(71) Applicant: Olympus Winter & Ibe GmbH, 22045 Hamburg (DE)
(72) Inventor: LAZZARI, Matteo, 20097 Hamburg (DE); JÜRGENS, Thorsten, 22045 Hamburg (DE)
(74) Representative: Weickmann & Weickmann PartmbB

(57) **Abstract**

The present invention relates to a medical camera system (100) for capturing images and processing said images to determine specific structures or conditions within a subject, comprising a camera module (102) configured to acquire images, and a control unit (104) operatively coupled to the camera module (102) and configured to receive and process output images, wherein the control unit (104) is further configured to detect at least one risk structure within the acquired images, detect the presence of at least one treatment device (200) and/or guiding laser beam within the acquired images, estimate a lateral thermal spread generated during activation of the at least one treatment device (200) in real-time based on settings and/or operational status of the at least one treatment device (200), evaluate a distance or position of a predefined iso-thermal line relative to the at least one detected risk structure, and cause at least one predetermined response when the distance between the at least one detected risk structure and the iso-thermal line falls below a predetermined threshold. The invention further relates to a medical installation, comprising such a camera system and at least one treatment device as well as to a use of such a system or installation and a corresponding method.

## Description

The present invention relates to a medical camera system for capturing images and processing said images to determine specific structures or conditions within a subject, as well as to a medical installation comprising such a camera system, the use of such a medical camera system or medical installation in endoscopic applications and a method for capturing images and processing said images to determine specific structures or conditions within a subject by means of a system or installation according to the invention.

In certain applications in the medical field, it is beneficial to be able to automatically and in real-time localize certain structures or conditions within the subject of a medical procedure in order to facilitate the work of a medical professional, such as a surgeon performing the procedure, by making corresponding information available to them by means of suitable display devices.

One particular application may be Otorhinolaryngology (ENT - Ear-Nose-Throat medicine) and specifically the identification of the posterior nasal nerve (PNN) during ENT procedures.

One frequently occurring condition in the field of Otorhinolaryngology is chronic Rhinosinusitis (CRS) which is characterized by the inflammation of the nasal cavities and sinuses that may persist for a minimum of 12 weeks or more. It is estimated that this disorder is affecting about 14 % of the global population, i.e. hundreds of millions of people. In the USA alone, it is for example estimated that around 16 million adults are suffering from said condition, with a higher prevalence in adults. Symptoms characterizing CRS are nasal congestion, facial pain or pressure, nasal discharge and reduced sense of smell. For the diagnosis of chronic Rhinosinusitis, specialized ENT doctors, often identified as Rhinologists, need to properly visualize the interior of the nasal passages and sinuses and a nasal endoscopy is typically recommended and performed by ENT doctors.

However, correct identification of the suspected area and nerve can be difficult based on optical information alone and thus further imaging tests such as CT scans or MRI may be performed to detect any structural abnormalities or chronic inflammation in the sinuses.

In particular in the past year, there has been growing interest in the treatment of the posterior nasal nerve and traditional surgical methods have frequently given way to alternative minimally invasive treatments. Nevertheless, several challenges persist in the realm of treatment area detection.

Treating the posterior nasal nerve (PNN) in chronic Rhinosinusitis (CRS) is essential in that it can help alleviate pain and improve overall quality of life for patients suffering from this condition. Despite advancements in imaging and endoscopy techniques, the diagnostic of chronic Rhinosinusitis (CRS) remains a challenging task for ENT doctors. In particular, detecting the posterior nasal nerve remains a challenge and often necessitates treatment in multiple spots or areas, which carries the risk of over-treatment and the creation of unnecessary lesions.

There are several methods to identify the PNN, including imaging, intranasal endoscopy (flexible or rigid), nerve stimulation and fluoroscopy, nevertheless, these techniques are infrequently employed during procedures and can be somewhat unwieldy. The task of promptly locating and treating the PNN with alternative methods such as RF-HF, ablation, cryoablation, laser therapy or other approaches remains a challenge during office-based procedures.

In particular, ENT doctors often encounter challenges when identifying and treating delicate anatomical structures, such as the posterior nasal nerve. The currently performed procedures may lack precision and real-time support which may lead to suboptimal treatment outcomes.

It is therefore the object of the present invention to provide an improved medical camera system, as well as a method for capturing images and processing said images to determine specific structures or conditions within a subject which may in particular be used for identification of the posterior nasal nerve during ENT procedures.

For this purpose, and in order to achieve the above-identified object, the medical camera system for capturing images and processing said images to determine specific structures or conditions within a subject according to the present invention comprises a camera module configured to acquire images, a control unit operatively coupled to the camera module and configured to receive and process output images, wherein the control unit is further configured to detect at least one risk structure within the acquired images, detect the presence of at least one treatment device and/or a guiding laser beam within the acquired images, estimate a lateral thermal spread generated during activation of the at least one treatment device in real-time based on settings and/or operational status of the at least one treatment device, evaluate a distance or position of a predefined iso-thermal line relative to the at least one detected risk structure, and cause at least one predetermined response when the distance between the at least one detected risk structure and the iso-thermal line falls below a predetermined threshold.

Herein, the acquisition of images by the camera module and subsequent processing thereof by the control unit may be performed at a given frame rate in the order of tens of Hz, wherein other modes of operation may also be provided such as a freeze frame feature to be triggered by the operator in order be able to examine the acquired images for an extended period of time.

While in the introduction above, specific reference has been made to the identification of the posterior nasal nerve (PNN) during ENT procedures, it is obvious that the medical camera system according to the present invention may also be used for capturing and processing images to determine specific structures or conditions within a subject in different fields of medicine and in the context of different treatments as well as diagnostic procedures. In particular, by means of the system according to the present invention, it becomes possible to pinpoint anatomical regions with precision, for example, identify nerves accurately, and execute diagnostics and treatment with minimal energy consumption, such as, for example, laser therapy or ablation, which reduces thermal stress on the tissue of the subject in the region of interest of the procedure. This integrated approach ensures the efficiency and success of medical interventions.

It shall be noted that in particular for the detection of the at least one risk structure within the acquired images and the detection of the presence of at least one treatment device and/or a guiding laser beam within the acquired images, the control unit may rely on different techniques and algorithms and in particular artificial intelligence (AI) methods, such as artificial neural networks. By means of employing suitable training data, such neural networks become capable of identifying the risk structures within the acquired images with high reliability and high precision and resolution. By then estimating the lateral thermal spread generated by the at least one treatment device in order to evaluate a distance or position of a predefined iso-thermal line relative to the risk structure, it becomes possible to give immediate feedback to a medical professional about potentially dangerous or harmful scenarios in which the risk structures would be subject to excessively high temperatures, which might damage the corresponding tissue.

In particular, the camera module may be configured to capture images via multiple light modalities, wherein preferably a first modality refers to standard white light and/or a second modality refers to near-infrared light. Relying on both white and infrared light allows for retrieving images with increased information content concerning the structures to be detected and identified in order to further improve the precision and reliability of the system according to the invention. Consequently, the images output by the camera module may thus comprise information in both a standard white light and a near-infrared light spectrum.

In order to be able to employ the system according to the present invention in standard medical procedures and environments, the system may further comprise an endoscopic camera head or an endoscope with which the camera module is provided. This allows for an improved handling of the system according to the invention by trained medical professionals which are accustomed to the use of endoscopes and endoscopic camera heads.

In order to be able to output information to the medical professional using the system according to the invention, it may further comprise a visual and/or audible alert system, wherein the at least one predetermined response may comprise the control unit causing the alert system to output a warning. Such visual and/or audible alert systems may be embodied in a known manner by sounds and visual effects informing the medical professional about the corresponding condition of the risk structure in the acquired images with respect to the predefined iso-thermal line, and in one example, an audible or visual signal may be provided once the iso-thermal line enters a predefined vicinity of the corresponding risk structure. The alert system may also be integrated with a display unit of the camera system according to the present invention in which the acquired images are presented for the medical professional using the system, for example by means of an overlay displaying additional information derived by the control unit.

Additionally or alternatively, the at least one predetermined response may comprise the control unit causing a deactivation of the at least one treatment device. In this way, once a condition is detected, in which the above discussed iso-thermal line reaches too close to an identified risk structure, the introduction of further heat into the subject through the operation of the treatment device may instantaneously be stopped in an automatic manner in order to prevent structural damage to the tissue of the risk structure. The previously discussed embodiments of an alert system and an automatic deactivation of the at least one treatment device may also be combined, for example, in that once the iso-thermal line in question reaches a first distance threshold to the risk structure, a visual and/or audible warning is output to the medical professional handling the system, while if the iso-thermal line subsequently moves beyond a second threshold distance which is shorter than the first threshold distance, an automatic deactivation of the at least one treatment device may be triggered as an automatic emergency measure.

In order to estimate the lateral thermal spread generated during activation of the least one treatment device, the settings and/or operational status of the at least one treatment device to be evaluated by the control unit may comprise at least one of energy levels and/or waveforms provided by an energy supply of the treatment device, the energy applied by the treatment device and the activation status of the treatment device. Thus, from these readily available information and data, the control unit is able to estimate the lateral thermal spread based on predefined algorithms and/or artificial intelligence methods which allow for a precise estimation of the lateral thermal spread.

Furthermore, the control unit may also be configured to detect tissue types and/or tissue structures within the subject in order to optimize the estimation of the thermal spread. For example, certain tissue types and/or tissue structures such as fatty tissues, arteries, veins, etc. may exhibit a different thermal conductivity than other tissue types and/or tissue structures, which may be considered by the control unit during the estimation of the thermal spread.

As already briefly mentioned above, the system according to the present invention may further comprise a display unit, wherein the control unit may further be configured to provide the acquired images together with an enhanced visualization overlay at the display unit. Such an enhanced visualization overlay may comprise arbitrary information which might be useful for the medical professional handling the system, such as highlighting the identified risk structures and/or treatment devices, drawing the estimated iso-thermal lines within the images, outputting warnings and/or color-coding the current conditions concerning the distance between the risk structures and the iso-thermal lines in question.

As has also already been mentioned above, the control unit of the system according to the invention may be configured to employ artificial intelligence, in particular for creating the enhanced visualization overlay and/or detecting the tissue types and/or structures, as well as the risk structures and the treatment device within the acquired images. For this purpose, known techniques in artificial intelligence and machine learning may be employed, in particular in the context of image processing and pattern recognition, while vast existing and available datasets of medical images and videos may be used as training data.

The system according to the invention may further comprise a graphical user interface, in particular for adjusting colors, shapes and other visual elements to display patient information and imaging data on a display unit of the system. In a highly integrated embodiment, the system may for example comprise a touchscreen display which both displays the acquired images together with additional enhanced information and may also serve as a graphical user touch interface by means of which the medical professional handling the camera system may adjust certain parameters of the display, such as magnification factors, color schemes and general information output settings.

The present invention also relates to a medical installation, comprising a camera system of the above-described type as well as at least one treatment device, wherein a control unit of the treatment device is integrated or operatively coupled with the control unit of the camera system. By integrating the camera system and the at least one treatment device concerning their control units, in particular the estimation of the lateral thermal spread based on settings and/or operational status of the treatment device becomes even more straightforward, since the necessary data can immediately be provided to the corresponding control unit actually performing the estimation. Also, different techniques concerning feedback between the camera system and the treatment device are facilitated, such as an automatic deactivation of the treatment device once an unwanted condition involving a detected risk structure and an estimated iso-thermal line is noticed.

In particular, the at least one treatment device of the installation according to the invention may comprise at least one of a monopolar or bipolar HF-applicator for ablation, a monopolar or bipolar cutting device, a cutting device using ultrasound energy, a cutting device using a combination of bipolar HF and ultrasound energy and a laser fiber. All of said types of treatment devices during their use spread heat into the subject in a predictable manner, such that the estimate of the lateral thermal spread of the heat generated by the treatment device during its use by the control unit of the medical camera system can be performed in a reliable manner.

In certain embodiments of an installation according to the present invention, the control unit of the camera system may in particular be configured to regulate the energy output of the at least one treatment device based on an energy utilization minimization scheme, such that the procedure performed by the medical professional using the installation is minimized to save energy and reduce the introduction of heat into the tissue of the subject.

As already discussed above, the medical camera system and medical installation according to the present invention may in particular be used in endoscopic applications, in particular diagnostic ENT applications.

Furthermore, the present application also relates to a method for capturing images and processing said images to determine specific structures or conditions within a subject by means of a system and installation according to the invention comprising the steps of acquiring images by means of the camera module, detecting at least one risk structure within the acquired images, detecting the presence of at least one treatment device and/or guiding laser beam within the acquired images, estimating a lateral thermal spread generated during activation of the at least one treatment device in real-time based on settings and/or operational status of the at least one treatment device, evaluating a distance or position of a predefined iso-thermal line relative to the at least one detected risk structure, and causing at least one predetermined response when the distance between the at least one detected risk structure and the iso-thermal line falls below a predetermined threshold.

It shall further be pointed out that the iso-thermal line may for example be predefined to be about 40°C. It shall also be pointed out that the features described in the context of certain embodiments of the system and installation according to the present invention can also be understood to relate to method steps of corresponding embodiments of methods according to the present invention. For example, in a similar manner as described above in the context of a system according to the invention, the method according to the invention may further comprise outputting a visual and/or audible alert as a predetermined response, a detection of tissue types and/or tissue structures within the subject, the provision of acquired images together with an enhanced visualization overlay, etc.

Further features and advantages of the present invention will become even clearer from the following description of embodiments thereof when viewed together with the accompanying drawings. These show in particular:
- Figure 1: a schematic view of a medical installation according to the present invention comprising a medical camera system according to the invention; and
- Figure 2: a flowchart of a method according to the present invention performed with the installation/system shown in figure 1.

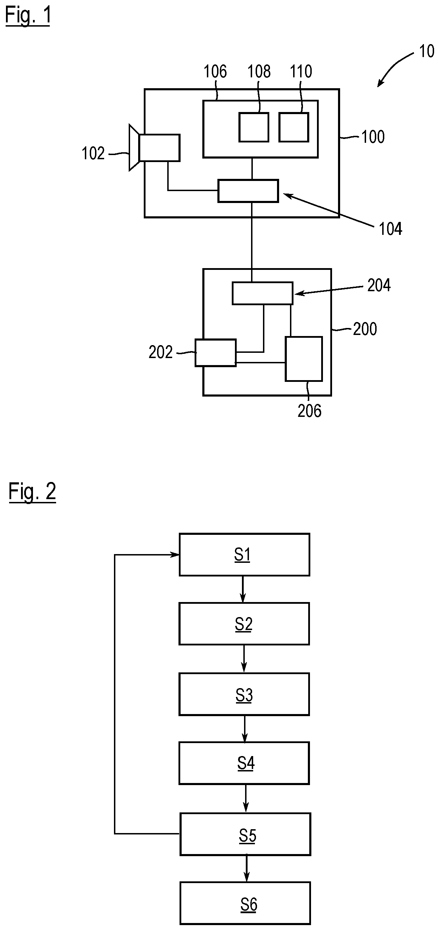

In figure 1, a medical installation according to the present invention is shown in a schematic view and generally denoted with reference numeral 10. Said medical installation 10 comprises a medical camera system 100 as well as a treatment device 200.

The medical camera system 100 comprises a camera module 102, which is configured to capture images via multiple light modalities, such as standard white light and near-infrared light, at one or more selectable framerates. The images acquired by the camera module 102 are output to a control unit 104, which may be formed by a known device, such as a microprocessor provided with a memory unit which serves to store software code as well as data necessary for performing the method steps discussed below.

The medical camera system 100 furthermore comprises a display unit 106 as well as a visual and/or audible alert system 108 wherein the display unit 106 and the alert system 108 may be integrated with one another and may also provide for a graphical user interface 110, which may be used for entering input commands, for example, for adjusting colors, shapes and other visual elements displayed. As such, the display unit 106, the alert system 108 and the graphical user interface 110 may be formed by a single touchscreen unit, optionally further comprising a speaker.

It shall further be pointed out that the camera module 102 may be provided at an endoscopic camera head or an endoscope in order to facilitate its use during medical procedures for capturing images within subjects.

The treatment device 200 on the other hand also comprises a control unit 204 in addition to an operational section 202, wherein said operational section may be adapted for ablation, cutting, providing a laser beam or similar applications. The control unit 204 of the treatment device may be operatively connected to or integrated with the control unit 104 of the camera system 100 in order to be able to exchange data and commands between the camera system 100 and the treatment device 200. In particular, the control unit 104 of the camera system 100 may receive data from the control unit 204 of the treatment device 200 concerning energy levels and a waveform provided by the energy supply 206 of the treatment device 200 and/or the energy applied by the treatment device 200 as well its activation status. Based on said data, the control unit 104 of the camera system 100 is able to estimate a lateral thermal spread generated during activation of the treatment device 202 and to evaluate the distance or position of a predefined iso-thermal line as a feature of the lateral thermal spread relative to at least one detected risk structure in accordance with the method described in the following with reference to figure 2.

According to the flowchart shown in figure 2, the camera module 102 of the medical camera system 100 in step S1 acquires images of regions of interest of a subject, for example with a certain frame rate (repetition rate) or on demand by the medical professional handling the installation. Said images are forwarded to the control unit 104 operatively coupled to the camera module 102 which is adapted to process said images, for example using artificial intelligence techniques and/or dedicated algorithms.

In this context, in step S2 at least one risk structure is detected within the acquired images, for example a nerve such as the posterior nasal nerve may be identified, while concurrently, the presence of the treatment device 200 is detected in the acquired images in step S3, also for example based on pattern recognition using artificial intelligence (AI) and/or other suitable algorithms.

Based on data provided by the control unit 204 of the treatment device 200 concerning the settings and/or operational status of the treatment device 200, the control unit 104 of the camera system 100 in step S4 estimates a lateral thermal spread generated by the treatment device 200 in real time, comprising the derivation of at least one iso-thermal line, for example representing 40°C. This step S4 may involve a detection and identification of tissue types and/or tissue structures within the subject in order to optimize the estimation of the thermal spread.

Based on these estimations, a distance or position of the predefined iso-thermal line relative to the at least one detected risk structure is evaluated in step S5, and if it is detected that the evaluated distance between the at least one risk structure and the iso-thermal line falls below a predetermined threshold, at least one predetermined response is caused in step S6, such as for example a visual and/or audible alert is output. Hereafter, the method is repeated starting at step S1.

It shall further be pointed out that the method according to the invention and described by means of steps S1 to S6 above are performed in the context of a visual output of the captured image with an enhanced information overlay on the display unit 106 of the medical camera system in which both the acquired images and additional information derived therefrom and from other sources are displayed for the medical professional handling the installation according to the invention. Therein, the output of the alert by means of the alert system 108 may also be included on the display 106, for example by means of a warning sign, blinking lights or similar means demanding the attention of the medical professional to the current condition and in particular the distance between the risk structure and the iso-thermal line in question.

## Claims

1. Medical camera system (100) for capturing images and processing said images to determine specific structures or conditions within a subject, comprising:
- a camera module (102) configured to acquire images; and
- a control unit (104) operatively coupled to the camera module (102) and configured to receive and process output images;
wherein the control unit (104) is further configured to:
- detect at least one risk structure within the acquired images;
- detect the presence of at least one treatment device (200) and/or guiding laser beam within the acquired images;
- estimate a lateral thermal spread generated during activation of the at least one treatment device (200) in real-time based on settings and/or operational status of the at least one treatment device (200);
- evaluate a distance or position of a predefined iso-thermal line relative to the at least one detected risk structure; and
- cause at least one predetermined response when the distance between the at least one detected risk structure and the iso-thermal line falls below a predetermined threshold.

2. System (100) according to claim 1,
wherein the camera module (102) is configured to capture images via multiple light modalities,
wherein preferably a first modality refers to standard white light and/or a second modality refers to the near-infrared light.

3. System (100) according to any of the preceding claims,
further comprising an endoscopic camera head or an endoscope at which the camera module is provided.

4. System (100) according to any of the preceding claims,
further comprising a visual and/or audible alert system (108), wherein the at least one predetermined response comprises the control unit causing (104) the alert system (108) to output a warning.

5. System (100) according to any of the preceding claims,
wherein the at least one predetermined response comprises the control unit (104) causing a deactivation of the at least one treatment device (200).

6. System (100) according to any of the preceding claims,
wherein the settings and/or operational status of the at least one treatment device (200) comprises at least one of energy levels and/or waveforms provided by an energy supply (206) of the treatment device (200), the energy applied by the treatment device (200) and the activation status of the treatment device (200).

7. System (100) according to any of the preceding claims,
wherein the control unit (104) is further configured to detect tissue types and/or tissue structures within the subject in order to optimize the estimation of the thermal spread.

8. System (100) according to any of the preceding claims,
further comprising a display unit (106);
wherein the control unit (104) is further configured to provide the acquired images together with an enhanced visualization overlay at the display unit (106).

9. System (100) according to any of the preceding claims,
wherein preferably the control unit (104) is configured to employ artificial intelligence, in particular for creating the enhanced visualization overlay and/or detecting the tissue types and/or structures.

10. System (100) according to any of the preceding claims,
further comprising a graphical user interface (110), in particular for adjusting colours, shapes and other visual elements to display patient information and imaging data.

11. Medical installation (10), comprising:
- a camera system (100) according to any of the preceding claims; and
- at least one treatment device (200),
wherein a control unit (204) of the treatment device (200) is integrated or operatively coupled with the control unit (104) of the camera system (100).

12. Installation (10) according to claim 11,
wherein the at least one treatment device (200) comprises at least one of a monopolar or bipolar HF-applicator for ablation, a monopolar or bipolar cutting device, a cutting device using ultrasound energy, a cutting device using a combination of bipolar HF and ultrasound energy and a laser fibre.

13. Installation (10) according to any of claims 11 and 12,
wherein the control unit (104) of the camera system (100) is configured to regulate energy output of the at least one treatment device (200) based on an energy utilization minimization scheme.

14. Use of a medical camera system (100) according to any of claims 1 to 10 or of a medial installation (10) according to any of claims 11 to 13 in endoscopic applications, in particular diagnostic ENT applications.

15. Method for capturing images and processing said images to determine specific structures or conditions within a subject by means of a system (100) according to any of claims 1 to 10 or an installation (10) according to any of claims 11 and 12, comprising the following steps:
- acquiring images by means of the camera module (S1);
- detecting at least one risk structure within the acquired images (S2);
- detecting the presence of at least one treatment device and/or guiding laser beam within the acquired images (S3);
- estimating a lateral thermal spread generated during activation of the at least one treatment device in real-time based on settings and/or operational status of the at least one treatment device (S4);
- evaluating a distance or position of a predefined iso-thermal line relative to the at least one detected risk structure (S5); and
- causing at least one predetermined response when the distance between the at least one detected risk structure and the iso-thermal line falls below a predetermined threshold (S6).
